# EUROPEAN PATENT APPLICATION

(11) **EP 4 767 959 A1**
(43) Date of publication of application: **01.07.2026**
(21) Application number: 25220734.5
(22) Date of filing: 04.12.2025
(51) Int. Cl.: A61B 18/02

(54) **CRYOABLATION PROBE WITH INTEGRATED VACUUM SLEEVE**

(30) Priority: 31.12.2024 US 202419006675
(71) Applicant: Varian Medical Systems, Inc., Palo Alto, CA 94304 (US)
(72) Inventor: FOSTER, Louis, Austin, 78717 (US)
(74) Representative: Mathisen & Macara LLP

(57) **Abstract**

A cryoprobe (100) includes a shell (102) extending in an axial direction (120) and forming a needle for positioning at a target tissue and a tube (108) extending in the axial direction positioned radially inward of the shell (102). An inner surface of the shell (102) and the outer surface of the tube (108) define an insulating cavity (304) therebetween.

## Description

### FIELD

The present disclosure relates to cryoablation probes that include an integrated vacuum sleeve.

### BACKGROUND

This section provides background information related to the present disclosure which is not necessarily prior art.

Systems and methods for providing cryoablation treatments may include cryoablation probes that are introduced at or near target tissue in a patient. A cryoablation system may include an extremely cold cryogen (liquid, gas, or mixed phase) that may be passed through a probe in thermal contact with the target tissue. Heat from the tissue passes from the tissue, through the probe, and into the cryogen that removes heat from the targeted tissue. This removal of heat causes tissue to freeze, resulting in the destruction of the targeted tissue. It is desirable that the cryogen is of sufficiently low temperature to quickly and efficiently cause the targeted tissues to freeze.

The cryoablation probe may include a needle that is inserted at or near the target tissue. The needle may include a vacuum sleeve that may insulate portions of the needle from the extreme low temperatures produced by the cryogen. The vacuum sleeve may prevent the needle from freezing or producing ice at undesired locations such as at or near healthy tissues. It may be desirable to maintain a small outer diameter for the needle so as to allow the needle to be positioned at or near a target tissue without damaging healthy tissues. Furthermore, smaller diameter needles may cause less trauma when the needle is inserted in the patient at the target tissue. There exists a need for improved cryoprobes and related methods that may produce freezing conditions at or near a target tissue without unnecessarily damaging healthy tissues.

### SUMMARY

This section provides a general summary of the disclosure, and is not a comprehensive disclosure of its full scope or all of its features.

In various embodiments of the present disclosure, a cryoprobe is provided as defined in the claims. The cryoprobe may include a shell extending in an axial direction and forming a needle for positioning at a target tissue and a tube extending in the axial direction positioned radially inward of the shell. An inner surface of the shell and the outer surface of the tube may define an insulating cavity therebetween.

In one aspect, the insulating cavity may form an insulating sleeve or an insulating layer. For example, the insulating cavity may form a vacuum sleeve or vacuum cavity to insulate the outer surface of the shell. The internal tube and the outer shell may form a vacuum sleeve along the length of the cryoprobe. The insulating layer may extend along a portion of the length of the needle of the cryoprobe. For example, the insulating layer may extend along a portion of the cryoprobe that is not located at or near an ice formation zone of the cryoprobe. The region of the shell at or near the tip may form an ice formation zone. Am internal lumen may supply cryogen through to the ice formation zone. The vacuum sleeve may be formed by an outer layer and an inner layer with a vacuum cavity positioned therebetween. In some embodiments, the outer layer is formed from the outer shell of the cryoprobe needle and the inner layer is formed by a tube sealed or connected to the outer shell at opposite ends.

In another aspect, the shell and the tube may have cylindrical shapes. The outer shell may be hollow to define an interior cavity of the shell. The internal tube may be positioned concentrically inside the outer shell. The outer shell and the internal tube are suitably co-axially arranged about the longitudinal axis of the needle. The outer diameter of the internal rube is suitable less than the internal diameter of the shell. The internal tube is suitably concentrically positioned within and spaced apart from the outer shell.

In another aspect, the insulating cavity may be a sealed cavity configured to maintain a vacuum.

In another aspect, a proximate end of the tube may include a mating surface configured to seal to the inner surface of the shell.

In another aspect, a diameter of the mating surface at the proximate end of the tube may be greater than a diameter of a central portion of the tube. The mating surface may be flared or enlarged compared to a central portion of the tube. The mating surface may include a gradual transition from the central portion to the mating surface. The outer diameter of the mating portion may be slightly smaller than an internal diameter of the shell so that the internal tube may be joined to the shell at the mating surface.

In another aspect, the mating surface of the tube may be connected to the inner surface of the shell.

In another aspect, the mating surface of the tube may be brazed to the inner surface of the shell. Alternatively, the shell and the internal tube may be connected together using other connections or other joining techniques such as crimping, adhesive, epoxy, welding, soldering, or the like.

In another aspect, a distal end of the tube may be axially separated from a distal end of the shell by a predetermined distance.

In another aspect, the predetermined distance may be configured to cause an iceball of a predetermined shape to be formed by the cryoprobe during use.

In another aspect, a distal end of the tube may include a joining surface configured to seal to the inner surface of the shell.

In another aspect, a diameter of the joining surface at the distal end of the tube may be greater than a diameter of a central portion of the tube. The joining surface may be a flared or widened portion of the internal tube.

In another aspect, the joining surface of the tube may be connected to the inner surface of the shell. The joining surface may have an outer diameter that is slightly less than the inner diameter of the shell so that the internal tube can be joined to the shell.

In another aspect, the joining surface of the tube may be brazed to the inner surface of the shell. Alternatively, the shell and the internal tube may be connected together using other connections or other joining techniques such as crimping, adhesive, epoxy, welding, soldering, or the like.

In another aspect, the tube may be positioned radially outward of a lumen that is configured to supply cryogen to the cryoprobe.

The thickness of the insulating cavity may be selected to provide a suitable insulation layer between the cryogen and the outer surface of the shell. In another aspect, a thickness of the insulating cavity may be in a range of about 0.02 mm to about 0.08 mm.

In another aspect, an outer diameter of the shell may be in a range of about 1.2mm to about 4.0 mm, preferably about 1.5 mm to about 2.5 mm.

In another aspect, a proximal end of the shell may be configured to connect to a handle of the cryoprobe.

In another aspect, a proximal end and a distal end of the tube may have an outer diameter that is different from an outer diameter of a central portion of the tube.

In another aspect, the insulating cavity may extend in the axial direction from a handle to a predetermined location relative to a tip of the cryoprobe.

In some embodiments of the present disclosure, a cryoprobe may include an outer shell extending in an axial direction forming a needle and configured to be positioned at a target tissue of a subject, a tip connected to a distal end of the outer shell, a handle connected to a proximal end of the outer shell opposite to the tip, a lumen positioned inside the outer shell and configured to supply cryogen to an ice formation zone of the needle at or near the tip, and an insulating tube inside the outer shell and radially outward of the lumen, the insulating tube joined to an inner surface of the outer shell at opposite ends to form a vacuum cavity between the inner surface of the outer shell and the outer surface of the insulating tube.

In another aspect, the vacuum cavity may be separated from the tip by a predetermined distance such that ice of a predetermined shape forms at the ice formation zone.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is a plan view of an example cryoablation needle in accordance with some embodiments of the present disclosure.
FIG. 2 is a side sectional view of the cryoablation needle of FIG. 1.
FIG. 3 is a magnified view of the portion labelled D of the cryoablation needle of FIG. 2.
FIG. 4 is a magnified view of the portion labelled C of the cryoablation needle of FIG. 2.

Corresponding reference numerals indicate corresponding parts throughout the several views of the drawings.

### DETAILED DESCRIPTION

Example embodiments will now be described more fully with reference to the accompanying drawings.

Example embodiments are provided so that this disclosure will be thorough and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

In various embodiments of the present disclosure, a cryoprobe is provided. The cryoprobe may be used to perform a cryoablation treatment in which a needle of the cryoprobe is inserted at or near a target tissue in a patient. A cryogen is supplied to the cryoprobe that causes heat to be removed from the area at or near the needle of the cryoprobe. Ice may form at the needle of the cryoprobe lowering the temperature of the target tissue to sufficient temperature for a predetermined period of time to destroy the target tissue.

The cryoprobes of the present disclosure include an insulating sleeve or insulating layer at portions of the needle of the cryoprobe. The insulating layer may insulate desired portions of the needle so that thermal transfer may be limited from portions of the cryoprobe. It may be desirable, for example, to limit heat transfer at portions of the cryoprobe that are not located at or near the target tissue. In addition, by limiting heat transfer at portions of cryoprobe, the iceball that is formed by operation of the cryoprobe can form at a predictable and/or desired size and shape.

It may further be desirable to minimize or reduce the size of the insulating layer in the cryoprobe. It may be desirable to use cryoprobe needles that may have an outer diameter in a range of about 2.5 mm to about 1.5 mm. In other examples, cryoprobe needles may have an outer diameter in a range of about 1.2 mm to about 4.0 mm. Such small size diameter cryoprobe needles may be desired to limit the impact of a cryoablation treatment on the patient. In addition, smaller diameter needles can be more easily guided and/or routed between bodily structures and reduce trauma to healthy tissues during a treatment.

The size of internal elements in the needle may impact the size of the overall or outer diameter of the cryoprobe. It may be desirable, therefore, to reduce the amount of elements or to reduce the size of the internal elements to maintain or reduce the overall outer diameter of the cryoprobe.

In various embodiments of the present disclosure, the cryoprobes may include an insulating layer formed as a vacuum sleeve or vacuum cavity positioned along a length of the cryoprobe needle. The vacuum sleeve may be formed by an outer layer and an inner layer with a vacuum cavity positioned therebetween. In some embodiments, the outer layer is formed from the outer shell of the cryoprobe needle and the inner layer is formed by a tube sealed or connected to the outer shell at opposite ends.

In some existing cryoprobes, a vacuum sleeve may be formed as a separate element from an inner and an outer tube. The vacuum sleeve may then be inserted or positioned inside the outer shell of the cryoprobe needle. In some embodiments of the present disclosure, the outer tube of the stand-alone vacuum sleeve may be eliminated thus reducing the amount of elements in the cryoprobe needle. This is an improvement over known or existing cryoprobes to allow simplification and reduction in the overall size. Still further, the reduction in elements and/or size of internal element may increase the volume inside the cryoprobe and may allow other elements to be positioned in the cryoprobe that otherwise would not be possible with existing designs.

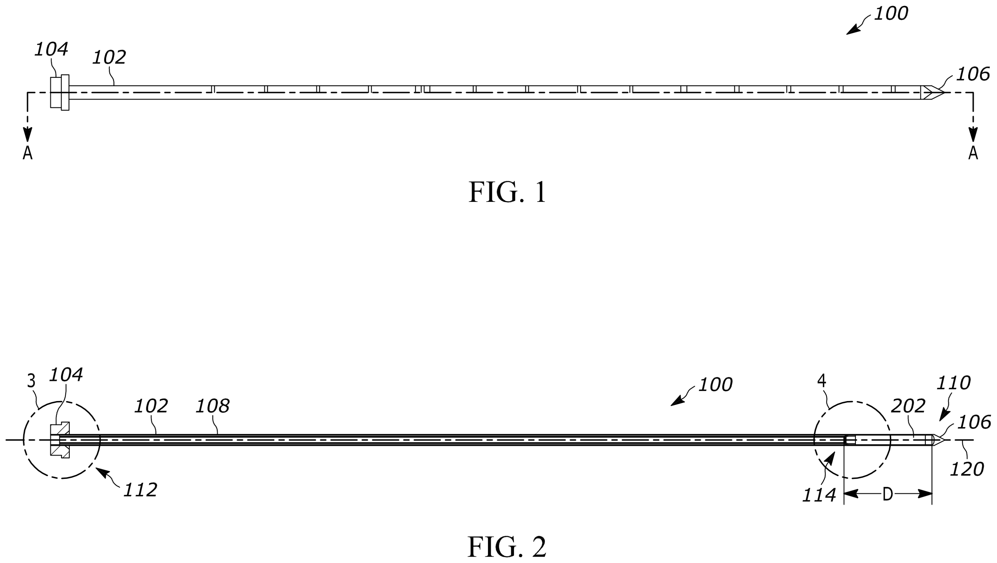

Turning now to FIGs. 1 and 2, an example cryoprobe 100 is illustrated. The cryoprobe 100 may include an outer shell 102, a safety washer 104, and a tip 106. The cryoprobe 100 may extend in an axial direction along an axis 120. The outer shell 102 may be a cylindrical member that may be hollow to allow the positioning of further elements in the interior cavity of the shell 102. The shell 102 may be made of various materials such as stainless steel or the like. In other example, other materials may be used.

As illustrated in FIG. 2, an internal tube 108 may be positioned inside the shell 102. The internal tube 108 may be a second cylindrical member of the cryoprobe 100 and may be positioned concentrically in the shell 102 along axis 120. In this example, the tube 108 may be connected to the shell 102 at a first or proximal end 112 and at a second or distal end 114. The tube 108 may have an outer diameter that is less than the internal diameter of the shell 102 to allow the tube to be positioned concentrically but spaced apart from the shell 102 to form an insulating cavity therebetween.

The second or distal end 114 of the tube 108 may be separated from the distal end 110 of the shell 102 or from the tip 106. The separation distance may be a predetermined distance D. The region of the shell 102 at or near the tip 106 may form an ice formation zone 202. An internal lumen (not shown) may supply a cryogen through the shell 102 to the ice formation zone 202. In some examples, the cryogen may exit the lumen in the ice formation zone and expand therein reducing the temperature of the cryogen to extremely low temperature levels. The cryogen may then flow away from the tip 106 of the shell 102 and remove thermal energy drastically dropping the temperature at the ice formation zone and producing an iceball at or around the ice formation zone 202. Such operation may freeze a target tissue of a patient.

The tube 108 and the shell 102 may form an insulating layer along a length of the cryoprobe 100. The insulating cavity located between an outer surface of the tube 108 and an inner surface of the shell 102 may maintain a vacuum in the cavity thus limiting transfer of thermal energy between a cryogen and the tissue located externally to the cryoprobe. The tube 108 and the shell 102 may form a vacuum sleeve along the length of the cryoprobe 100.

As can be appreciated, the predetermined distance D may affect a size or shape of an iceball that is produced at the ice formation zone 202. The distance D may be selected or determined, for example, based on whether a spherical or ellipsoidal iceball is desired and/or based on a desired diameter of the iceball. For example, as the distance D is reduced (i.e., the distal end 114 of the tube 108 is positioned closer to the tip 106), the diameter of a produced iceball may be reduced). Conversely, as the distance D is increased (i.e., the distal end 114 of the tube 108 is positioned further away from the tip 106), the diameter of the iceball may be increased and/or the iceball may have a more ellipsoidal shape with major axis of the ellipsoid positioned along axis 120.

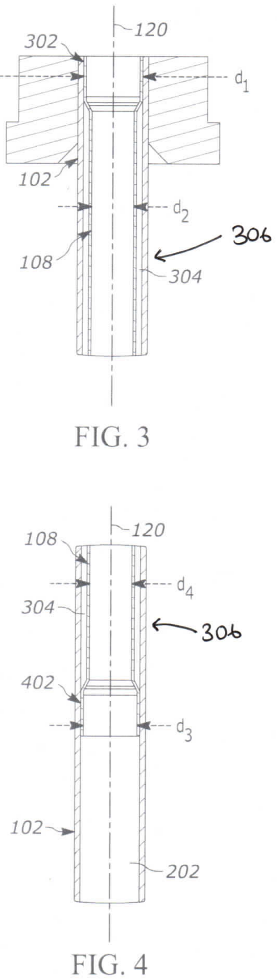

Turning now to FIGs. 3 and 4, the proximate end 112 and the distal end 114 of the tube 108 are shown in further detail. In some embodiments, the proximate end 112 of tube 108 may include a mating surface 302. The mating surface 302 may be flared or enlarged to have an outer diameter d1 that is greater than an outer diameter d2 of a central portion 306 of tube 108. The mating surface 302 may include a gradual transition from the central portion 306 to the mating surface 302. The diameter d1 of the mating portion may be slightly smaller than an internal diameter of the shell 102 so that the tube 108 may be joined to the shell 102. In one example, the tube 108 is brazed to the shell 102 at the mating surface 302.

The distal end 114 of the tube 108 may include a joining surface 402. The joining surface 402 may be shaped in a similar manner to the mating surface 302 described above. The joining surface 402 may be a flared or widened portion of the tube 108. The joining surface 402 may have an outer diameter d3 that is greater than an outer diameter d4 of the central portion 306 of tube 108. The diameter d4 of the central portion 306 may be the same as the diameter d2 of central portion 306 shown in FIG. 3. The joining surface 402 may have a diameter d3 that is slightly less than the inner diameter of the shell 102 so that the tube 108 can be joined to the shell 102. The joining surface 402 may be brazed to the inner surface of the shell 102.

The relative diameters of the central portion 306 of the tube 108 and the shell 102 may be selected to have a suitable thickness of an insulating cavity 304. In some examples, the thickness of the insulating cavity (i.e., the size of the gap between the outer surface of the tube 108 and the inner surface of the shell 102) may be in a range of about 0.02 mm to about 0.20 mm. In other examples, other thicknesses may be used. The thickness of the insulating cavity may be selected to provide a suitable insulation layer between the cryogen and the outer surface of the shell 102. The thickness of the insulating cavity may also be sufficient such that when limited bending of the shell 102 occurs during insertion of the cryoprobe 100, that the tube 108 does not contact the shell 102 to cause conduction of thermal energy thus defeating the insulative properties of the insulating gap 304.

In some embodiments, the insulating cavity 304 may be a vacuum cavity formed between the tube 108 and the shell 102. The tube 108 may be joined to the shell 102 in a vacuum furnace, in some examples. The tube 108 may be positioned in the shell 102 with brazing material and heated under vacuum until such temperature that the brazing material is wicked into the joint at the mating surface 302 and the joining surface 402. The joints between the tube 108 and the shell 102 may be completely sealed such that a vacuum condition is maintained in the vacuum cavity 304 when the cryoprobe 100 is removed from the vacuum furnace.

In other examples, the cryoprobe 100 may have other configurations and/or connections between the shell 102 and the tube 108. In other examples, the shell 102 and the tube 108 may be connected together using other connections or other joining techniques such as crimping, adhesive, epoxy, welding, soldering, or the like. In yet other examples, the tube 108 may have a substantially constant outer diameter and the shell 102 may be necked or swaged to have a reduced diameter at the location of connection between the shell 102 and the tube 108.

While not shown in the figures, the cryoprobe 100 may be an element of complete cryoprobe assembly. The cryoprobe 100 may be connected to a handle. The safety washer 104 may be inserted into a straight or a right-angle handle. The cryoprobe 100 may also be connected to one or more cryogen lines to supply and/or return cryogen. The cryoprobe 100 may also be connected to one or more control lines that may transfer signals from or to sensors of the cryoprobe 100 or from or to other elements of the cryoprobe 100, such as heaters.

The following is a list of non-limiting illustrative embodiments disclosed herein:
Illustrative embodiment 1: A cryoprobe comprising: a shell extending in an axial direction and forming a needle for positioning at a target tissue; and a tube extending in the axial direction positioned radially inward of the shell; wherein an inner surface of the shell and the outer surface of the tube define an insulating cavity therebetween.
Illustrative embodiment 2: The cryoprobe of illustrative embodiment 1, wherein the insulating cavity forms a vacuum sleeve to insulate the outer surface of the shell.
Illustrative embodiment 3: The cryoprobe of any of illustrative embodiment 1 or 2, wherein the shell and the tube comprise cylindrical shapes.
Illustrative embodiment 4: The cryoprobe of any of illustrative embodiments 1 to 3, wherein the insulating cavity comprises a sealed cavity configured to maintain a vacuum.
Illustrative embodiment 5: The cryoprobe of any of illustrative embodiments 1 to 4, wherein a proximate end of the tube comprises a mating surface configured to seal to the inner surface of the shell.
Illustrative embodiment 6: The cryoprobe of illustrative embodiment 5, wherein a diameter of the mating surface at the proximate end of the tube is greater than a diameter of a central portion of the tube.
Illustrative embodiment 7: The cryoprobe of any of illustrative embodiments 5 or 6, wherein the mating surface of the tube is connected to the inner surface of the shell.
Illustrative embodiment 8. The cryoprobe of any of illustrative embodiments 5 to 7, wherein the mating surface of the tube is brazed to the inner surface of the shell.
Illustrative embodiment 9: The cryoprobe of any of illustrative embodiment 1 to 8, wherein a distal end of the tube is axially separated from a distal end of the shell by a predetermined distance.
Illustrative embodiment 10: The cryoprobe of illustrative embodiment 9, wherein the predetermined distance is configured to cause an iceball of a predetermined shape to be formed by the cryoprobe during use.
Illustrative embodiment 11: The cryoprobe of any of illustrative embodiments 1 to 10, wherein a distal end of the tube comprises a joining surface configured to seal to the inner surface of the shell.
Illustrative embodiment 12: The cryoprobe of illustrative embodiment 11, wherein a diameter of the joining surface at the distal end of the tube is greater than a diameter of a central portion of the tube.
Illustrative embodiment 13: The cryoprobe of any of illustrative embodiments 11 or 12, wherein the joining surface of the tube is connected to the inner surface of the shell.
Illustrative embodiment 14: The cryoprobe of any of illustrative embodiments 11 to 13, wherein the joining surface of the tube is brazed to the inner surface of the shell.
Illustrative embodiment 15: The cryoprobe of any of illustrative embodiments 1 to 14, wherein the tube is positioned radially outward of a lumen that is configured to supply cryogen to the cryoprobe.
Illustrative embodiment 16: The cryoprobe of any of illustrative embodiments 1 to 15, wherein a thickness of the insulating cavity is in a range of about 0.02 mm to about 0.08 mm.
Illustrative embodiment 17: The cryoprobe of any of illustrative embodiments 1 to 16, wherein an outer diameter of the shell is in a range of about 1.5 mm to about 2.5 mm.
Illustrative embodiment 18: The cryoprobe of any of illustrative embodiments 1 to 17, wherein a proximal end of the shell is configured to connect to a handle of the cryoprobe.
Illustrative embodiment 19: The cryoprobe of any of illustrative embodiments 1 to 18, wherein a proximal end and a distal end of the tube have an outer diameter that is different from an outer diameter of a central portion of the tube.
Illustrative embodiment 20: The cryoprobe of any of illustrative embodiments 1 to 19, wherein the insulating cavity extends in the axial direction from a handle to a predetermined location relative to a tip of the cryoprobe.
Illustrative embodiment 21: A cryoprobe comprising: an outer shell extending in an axial direction configured to be positioned at a target tissue of a subject; a tip connected to a distal end of the outer shell; a handle connected to a proximal end of the outer shell opposite to the tip; a lumen positioned inside the outer shell and configured to supply cryogen to an ice formation zone of the needle at or near the tip; and an insulating tube inside the outer shell and radially outward of the lumen, the insulating tube joined to an inner surface of the outer shell at opposite ends to form a vacuum cavity between the inner surface of the outer shell and the outer surface of the insulating tube.
Illustrative embodiment 22: The cryoprobe of illustrative embodiment 21, wherein the vacuum cavity is separated from the tip by a predetermined distance such that ice of a predetermined shape forms at the ice formation zone.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A cryoprobe (100) comprising:
a shell (102) extending in an axial direction (120) and forming a needle for positioning at a target tissue; and
a tube (108) extending in the axial direction (120) positioned radially inward of the shell (102);
wherein an inner surface of the shell and the outer surface of the tube define an insulating cavity (304) therebetween.

2. The cryoprobe of claim 1, wherein the insulating cavity (304) forms a vacuum sleeve to insulate the outer surface of the shell (102).

3. The cryoprobe of claim 1, wherein the shell and the tube comprise cylindrical shapes.

4. The cryoprobe of claim 1, 2 or 3, wherein the insulating cavity (304) comprises a sealed cavity configured to maintain a vacuum.

5. The cryoprobe of any one of the preceding claims , wherein a proximate end (112) of the tube comprises a mating surface (302) configured to seal to the inner surface of the shell (102), and optionally a diameter (d₁) of the mating surface (302) at the proximate end (112) of the tube is greater than a diameter (d₂) of a central portion (306) of the tube, and optionally the mating surface (302) of the tube (108) is connected to the inner surface of the shell (102), and optionally the mating surface (302) of the tube is brazed to the inner surface of the shell (102).

6. The cryoprobe of any one of the preceding claims, wherein a distal end (114) of the tube (108) is axially separated from a distal end (110) of the shell (102) by a predetermined distance (D).

7. The cryoprobe of claim 6, wherein the predetermined distance (D) is configured to cause an iceball of a predetermined shape to be formed by the cryoprobe (100) during use.

8. The cryoprobe of any one of the preceding claims, wherein a distal end (114) of the tube (108) comprises a joining surface (402) configured to seal to the inner surface of the shell (102) and optionally a diameter (d₃) of the joining surface (402) at the distal end (114) of the tube (108) is greater than a diameter (d₄) of a central portion (306) of the tube (108), ad optionally the joining surface (402) of the tube (108) is connected to the inner surface of the shell (102), and optionally the joining surface (402) of the tube is brazed to the inner surface of the shell (102).

9. The cryoprobe of any one of the preceding claims, wherein the tube (108) is positioned radially outward of a lumen that is configured to supply cryogen to the cryoprobe.

10. The cryoprobe of any one of the preceding claims, wherein a thickness of the insulating cavity (304) is in a range of about 0.02 mm to about 0.08 mm.

11. The cryoprobe of any one of the preceding claims, wherein an outer diameter of the shell is in a range of about 1.5 mm to about 2.5 mm.

12. The cryoprobe of any one of the preceding claims, wherein a proximal end (112) and a distal end (114) of the tube (108) have an outer diameter that is different from an outer diameter of a central portion (306) of the tube (108).

13. The cryoprobe of any one of the preceding claims, wherein the insulating cavity (304) extends in the axial direction from a handle to a predetermined location relative to a tip (106) of the cryoprobe (100).

14. A cryoprobe (100) comprising:
an outer shell (102) extending in an axial direction (120) forming a needle and configured to be positioned at a target tissue of a subject;
a tip (106) connected to a distal end (110) of the outer shell (102);
a handle connected to a proximal end (112) of the outer shell (102) opposite to the tip (106);
a lumen positioned inside the outer shell and configured to supply cryogen to an ice formation zone of the needle at or near the tip (102); and
an insulating tube (108) inside the outer shell (102) and radially outward of the lumen, the insulating tube (108) joined to an inner surface of the outer shell at opposite ends to form a vacuum cavity (304) between the inner surface of the outer shell (102) and the outer surface of the insulating tube (108).
